# EUROPEAN PATENT SPECIFICATION

(11) **EP 2 856 992 B1**
(45) Date of publication and mention of the grant of the patent: **04.04.2018**
(21) Application number: 13800748.9
(22) Date of filing: 03.06.2013
(51) Int. Cl.: A61F 13/15, A61F 13/49

(54) **DISPOSABLE DIAPER**
WEGWERFWINDEL
COUCHE JETABLE

(30) Priority: 04.06.2012 JP 2012127390
(43) Date of publication of application: 08.04.2015
(73) Proprietor: Unicharm Corporation, Ehime 799-0111 (JP)
(72) Inventor: KATSURAGAWA, Kunihiko, Kanonji-shi Kagawa 769-1602 (JP); SASAYAMA, Kenichi, Kanonji-shi Kagawa 769-1602 (JP)
(74) Representative: Dolleymores
(86) International application number: PCT/JP2013/065402
(87) International publication number: WO 2013/183602

(56) References cited:
- WO-A1-01/39714
- WO-A1-2004/037146
- WO-A1-2011/001944
- JP-A- H03 136 653
- JP-A- H07 184 947
- JP-A- H09 509 350
- JP-A- 2001 137 281
- JP-A- 2006 081 653
- JP-A- 2008 104 873
- JP-A- 2008 104 873
- JP-A- 2011 139 857
- JP-A- 2011 250 878
- JP-A- 2012 050 634
- JP-A- 2013 078 369
- US-A1- 2010 280 478

## Description

### {Technical Field}

The present invention relates to disposable diapers.

### {Background}

Conventionally, disposable wearing articles having a pocket adapted to receive body exudates in at least one of front and rear waist regions are well known. For example, JP H7-184947 A discloses a disposable diaper including a liquid-absorbent structure, barrier-cuffs located along lateral edges of a waist barrier sheet region, and panels provided in the front and rear waist regions and extending between the barrier-cuffs so as to cross the liquid-absorbent structure.

### {Citation List}

### {Patent Literature}

{PTL 1}: JP H7-184947 A

### {Summary}

### {Technical Problem}

According to the disclosure of JP H7-184947 A, in the disposable diaper disclosed therein, the barrier-cuffs can prevent body exudates from leaking sideways and the pocket defined between the waist barrier sheet and the liquid-absorbent structure may receive body exudates flowing toward the front and rear waist regions.

However, in such a diaper, the barrier-cuffs have proximal edges located inboard of the lateral edges of the diaper and, in the crotch region, a distance dimension of the barrier-cuffs in a transverse direction is smaller than a dimension of the crotch region in the transverse direction. Consequently, when a relatively large amount of body exudates is voided, an excess amount of body exudates might overflow the barrier-cuffs. Meanwhile, the waist pocket sheets are attached to respective distal edges of the barrier-cuffs and the pocket adapted to receive the body exudates are relatively small. Consequently, a relatively large amount of body exudates may not be received or contained.

An obj ect of the present invention is to provide a disposable diaper provided at least in one of the front and rear waist regions with a pocket adapted to receive and to contain a relatively large amount of body exudates.

A further prior art arrangement is known from WO 2011/001944.

### {Solution to Problem}

The present invention relates to a disposable diaper as recited by Claim 1.

### {Advantageous Effects of Invention}

In the disposable diaper disclosed in the present invention, the chassis has the lateral elastic regions formed by folding the lateral portions and, in the rear waist region, the waist pocket panel is attached to the inner lateral edge portions of the lateral elastic regions and the elastic belt panels are attached to the outer lateral portions so that, during use of the diaper, the lateral elastic regions may be spaced away from the mainportion of the chassis and a relatively large body exudates receiving space may be formed in the rear waist region and the crotch region.

### {Brief Description of Drawings}

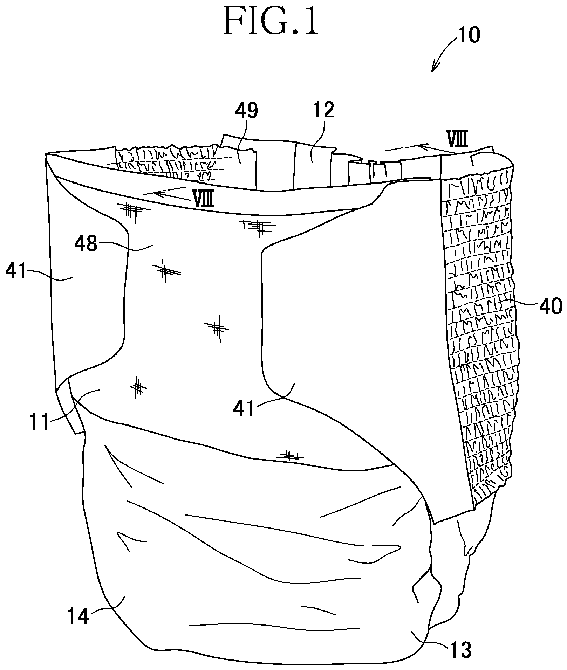

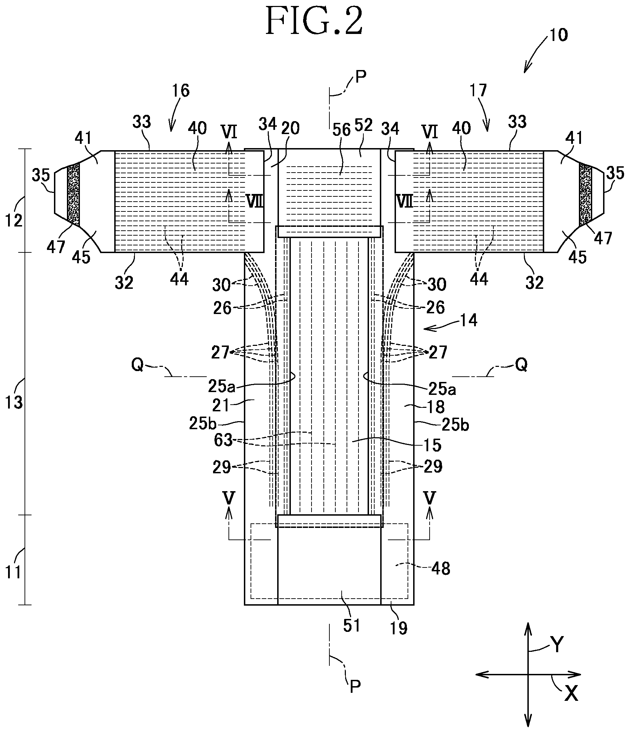

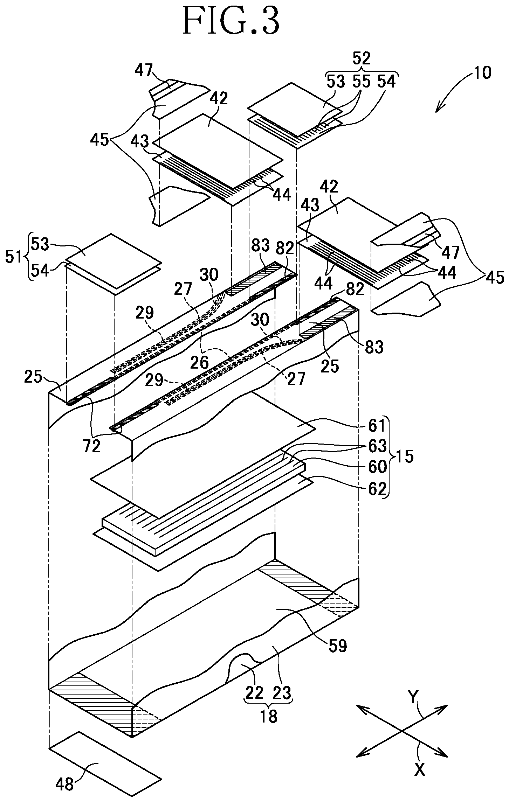

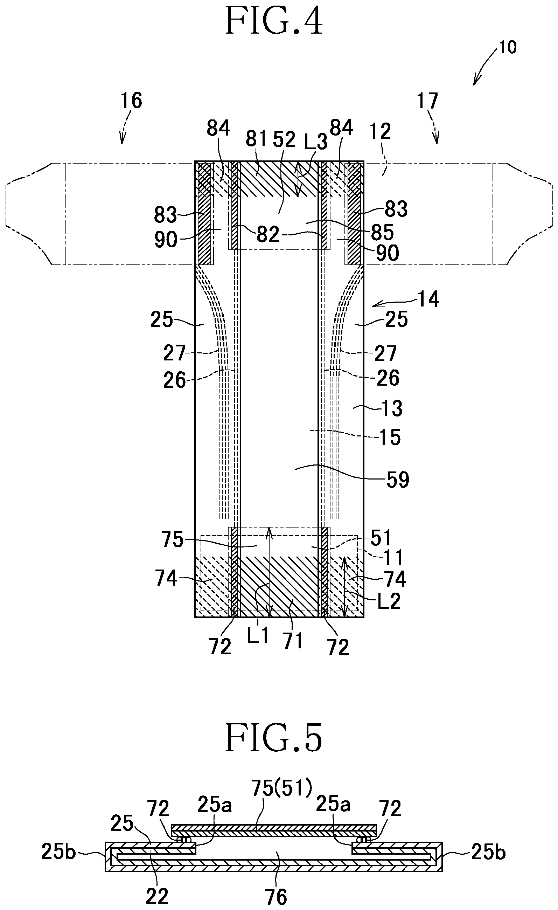

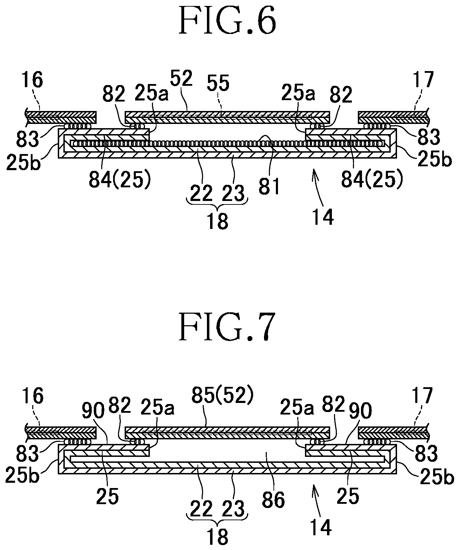

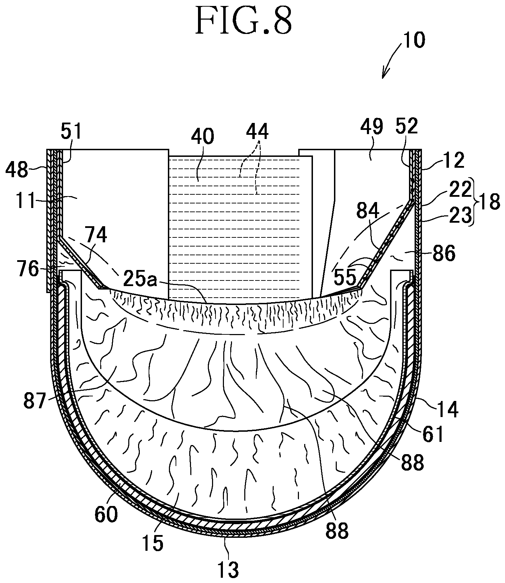

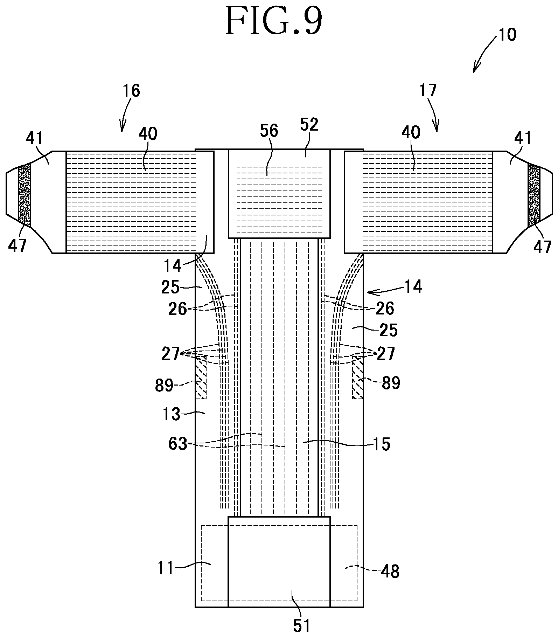

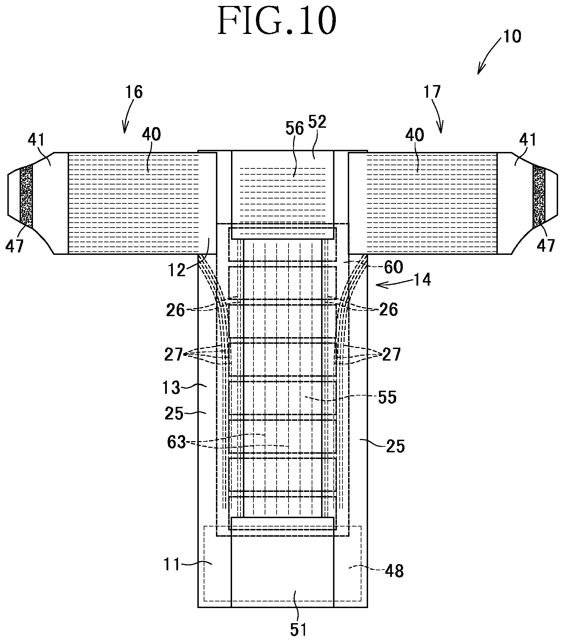

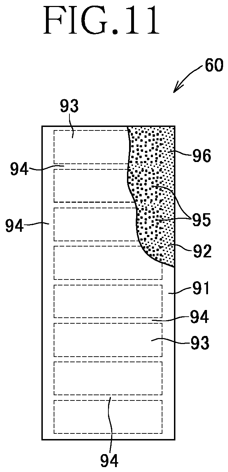

{Fig. 1} Fig. 1 is a perspective view of a disposable diaper according to a first embodiment of the present invention.
{Fig. 2} Fig. 2 is a developed plan view of the diaper developed in a front-back direction after respective associated mechanical fasteners have been disengaged from each other.
{Fig. 3} Fig. 3 is an exploded perspective view of the diaper.
{Fig. 4} Fig. 4 is a developed plan view of the diaper, illustrating a chassis and respective joint regions but deleting a liquid-absorbent structure.
{Fig. 5} Fig. 5 is a schematic sectional view taken along line V-V in Fig. 2.
{Fig. 6} Fig. 6 is a schematic sectional view taken along line VI-VI in Fig. 2.
{Fig. 7} Fig. 7 is a schematic sectional view taken along line VII-VII in Fig. 2.
{Fig. 8} Fig. 8 is a schematic sectional view taken along line VIII-VIII in Fig. 1.
{Fig. 9} Fig. 9 is a developed plan view similar to Fig. 2, illustrating a second embodiment.
{Fig. 10} Fig. 10 is a developed plan view similar to Fig. 2, illustrating a third embodiment.
{Fig. 11} Fig. 11 is a partially cutaway developed plan view of the liquid-absorbent structure according to the third embodiment.

### {Description of Embodiments}

### <First Embodiment>

Referring to Figs. 1 through 4, a disposable diaper 10 according to this embodiment has a longitudinal direction Y, a transverse direction X which is orthogonal to the longitudinal direction Y, a longitudinal axis P-P and a transverse axis Q-Q. The diaper 10 includes a chassis 14 having a skin-facing surface, a non-skin-facing surface, a front waist region 11, a rear waist region 12 and a crotch region 13 extending in the longitudinal direction Y between the front and rear waist regions 11, 12; a liquid-absorbent structure 15 attached to the skin-facing surface at least in the crotch region 13; and first and second elastic belt panels 16, 17 extending outwardly in the transverse direction X from opposite lateral edges of the rear waist region 12 of the chassis 14.

The chassis 14 has a longitudinally long, generally rectangular configuration and has an outer cover 18, a front end portion 19 lying in the front waist region 11, a rear end portion 20 lying in the rear waist region 12 and an intermediate portion 21 lying in the crotch region 13. The outer cover 18 has interior and exterior sheets 22, 23 each formed of a liquid-impermeable fibrous nonwoven fabric, a plastic film or a laminate sheet thereof. The interior and exterior sheets 22, 23 are joined to each other with a hot melt adhesive (not shown) distributed to an inner surface of one of these two sheets 22, 23. Opposite lateral portions of the outer cover 18 are folded inwardly to form a pair of lateral elastic regions 25 extending in the longitudinal direction Y in the crotch region 13. The lateral elastic regions 25 respectively have inner lateral edge portions 25a and outer lateral portions 25b corresponding to the folded portions of the outer cover 18.

The respective lateral elastic regions 25 are provided with a plurality of thread, strand or string first and second leg elastics 26, 27 extending in the longitudinal direction Y and whereby the lateral elastic regions 25 are elasticized at least in the crotch region 13. The first leg elastics 26 rectilinearly extend in the longitudinal direction Y along respective inner lateral edge portions 25a of the lateral elastic regions 25. The second leg elastics 27 lie outboard of the respective first leg elastics 26 in the transverse direction X and have rectilinear portions 29 extending from the transverse axis Q-Q toward the side of the front waist region 11 and curved portions 30 extending from the transverse axis Q-Q toward the rear waist region 12. The curved portions 30 gradually extend outwardly in the transverse direction X as these curved portions 30 extend rearwardly. The first and second leg elastics 26, 27 are contractibly attached in the longitudinal direction Y between the interior and exterior sheets 22, 23 with a hot melt adhesive (not shown) distributed to the inner surface of one of these interior and exterior sheets 22, 23.

Each of the elastic belt panels 16, 17 has end edges 32, 33 spaced apart from and opposed to each other in the longitudinal direction Y and inner and outer lateral edges 34, 35 spaced apart from and opposed to each other in the transverse direction X. The inner lateral edges 34 rectilinearly extend in the longitudinal direction Y and the outer lateral edges 35 are convex outwardly in the transverse direction X. The inner lateral edges 34 of the respective elastic belt panels 16, 17 are attached to the chassis 14 through respective third joint regions 83 extending along the outer edges 25b of the respective lateral elastic regions 25 as will be described later. The elastic belt panels 16, 17 further include first elastic portions 40 which are stretchable and contractible in the transverse direction X and tabs 41 lying outboard of the associated first elastic portions 40 as viewed in the transverse direction X.

Each of the first elastic regions 40 includes a first sheet 42 formed of a fibrous nonwoven fabric or a plastic sheet and lying on the side of the skin-facing surface, a second sheet 43 lying on the side of the non-skin-facing surface and a plurality of thread, strand or string first waist elastics 44 extending in the transverse direction X. The first waist elastics are contractibly attached under tension between the first and second sheets 42, 43 in the transverse direction X with a hot melt adhesive. Each of the tabs 41 is formed of generally trapezoidal sheet materials 45. The sheet materials 45 may be composed of, for example, formed of fibrous nonwoven fabrics or plastic sheets. These two sheet materials are respectively joined to the first sheet 42 and the second sheet 43 with an adhesive or by fusion bonding and the outer side portion of the first elastic region 40 is secured between the sheet materials 45.

Outer end portions of the respective sheet materials 45 are provided on the skin-facing surfaces thereof with first fastening elements 47 each formed of sheet material having a relatively high stiffness and tensile strength and made from, for example, aplastic film, a fibrous nonwoven fabric or a laminate thereof or craft paper and including a multiplicity of hooks of a mechanical fastener. Referring to Fig. 1, the front waist region 11 is provided on its entire exterior surface with second fastening element 48 formed of sheet material having a relatively high stiffness and tensile strength made from, for example, a plastic film, a fibrous nonwoven fabric, a laminate thereof or craft paper and including a multiplicity of loops of a mechanical fastener. The first fastening elements 47 may be detachably engaged with the second fastening element 48 to define a waist-opening 49 and a pair of leg-openings.

The second fastening element 48 is provided on the entire exterior surface of the front waist region 11 and a range to be targeted by the first fastening element 47 is sufficiently large to facilitate a size adjustment of the diaper put on the wearer's body. While the diaper is usually put on the wearer's body lying on the back, the diaper 10 according to the present invention may be rather easily put on the wearer's body even when the wearer is in an upright posture. Specifically, a helper or a care person may hold the front waist region 11 against the wearer's entire abdominal region with the one hand and simultaneously put the first fastening elements 47 into engagement with the second fastening element 48 with the other hand.

Between the inner lateral edge portions 25a of the lateral elastic regions 25 in the front and rear waist regions 11, 12, waist pocket panels 51, 52 are provided so as to cross the liquid-absorbent structure 15. Each of the waist pocket panels 51, 52 includes an interior sheet 53 formedof a liquid-impermeable SMS (spunbonded/meltblown/spunbonded) fibrous nonwoven fabric or a spun bonded nonwoven fabric having a mass per unit area in a range of about 5 to about 15g/m² and an exterior sheet 54 formed of a breathable plastic sheet.

The waist pocket panel 52 lying in the rear waist region 12 additionally includes a plurality of thread, strand or string second waist elastics 55 interposed between the interior and exterior sheets 53, 54. The second waist elastics 55 are contractibly attached between the interior and exterior sheets 53, 54 with a hot melt adhesive. The waist pocket panel 52 has a second elastic region 56 defined by the region in which the second waist elastics 55 are arranged and elasticized at least in the transverse direction X.

The first and second leg elastics 26, 27 may be formed of a thread, strand or string elastic material each having a fineness in a range of about 470 to about 940 dtex and a tensile ratio in a range of about 2.0 to about 2.8, the first waist elastics 44 may be formed of a thread, strand or string elastic material each having a fineness about 470 to 940 dtex and a tensile ratio in a range of about 2.5 to about 3.0, and the second waist elastics 55 may be formed of a thread, strand or string elastic material each having a fineness in a range of about 470 to about 940 dtex and a tensile ratio in a range of about 2.0 to about 2.5. In the diaper 10 developed in the longitudinal direction Y and the transverse direction X, a dimension of the first waist elastics 44 in the transverse direction X, i.e., a dimension of the respective first elastic regions 40 in the transverse direction X is larger than a dimension of the second waist elastics 55 in the transverse direction X, i.e., a dimension of the first elastic region 56 in the transverse direction X. Specifically, the former's dimension is in a range of about 190 to about 230 mm and the latter's dimension is in a range of about 120 to about 160 mm. The dimension of the respective first elastic regions 40 in the transverse direction X may be set to be larger than that of the dimension of the second elastic region 56 in the transverse direction X in this manner to improve the fit about the wearer's waist, thereby preventing the crotch region from being displaced during use of the diaper 10. A tensile strength (designated hereunder as "tensile stress") per unit area of the respective first elastic regions 40 is preferably higher than a tensile stress per unit area of the second elastic region 56. More specifically, based on test pieces each having a dimension of 25 mm in the longitudinal direction Y cut out from the respective regions, a tensile stress of the first elastic regions 40 at 80% of the maximum elongation is about 1.3 N and a tensile stress of the second elastic region 56 at 80% of the maximum elongation is about 1.0 N. The relationship established in such a manner that the tensile stress of the second elastic region 56 is lower than the tensile stress of the respective first elastic regions 40 ensures that, when the diaper 10 is put on the wearer's body and the first and second elastic regions 40, 56 are circumferentially stretched about the wearer's waist, the second elastic region 56 is stretched at a degree higher than a degree at which the first elastic regions 40 are stretched and, in consequence, the waist pocket panel 52 stably fits about the wearer's waist.

### <Measuring method for tensile stress of respective elastics>

Either one of the elastic belt panels 16, 17 and either one of the waist pocket panels 51, 52 were taken out from the diaper 10 and pieces each having a width dimension of about 25 mm and a dimension of about 160 mm were cut out of the elastic belt panels and the connecting sheet as test pieces. Sheet panels each having a width dimension of about 30 mm were attached to both ends of the respective test pieces so that the test piece may be secured in a contractile state between chucks (initial inter-chuck distance is 30 mm and may be appropriately adjusted depending on the test piece) of Tensile Tester (manufactured by Shimadzu Corporation in Japan). Then the test piece was elongated at a pulling rate of 200 mm/min and a load (N) at a moment of 80% of the maximum elongation was measured and tensile stress of the respective test pieces was calculated according to the following formula: Tensile stress = measured value (N) ÷ region's width (mm) . Such measurement was repeated three times for each of the test pieces and average values were obtained as tensile stresses of the first elastic regions 40 and of the second elastic region 56, respectively.

In the rear waist region 12, the first and second elastic regions 40, 50 contract in the transverse direction X and consequently the front and rear waist regions 11, 12 in the state of being joined to each other fit about the wearer's waist with high stability. With the arrangement according to this embodiment such that the waist pocket panel 52 and the elastic belt panels 16, 17 are separately formed and the first elastic regions 40 and the second elastic region 56 are spaced apart from each other in the rear waist region 12, the first leg elastics 26 are pulled outwardly in the transverse direction X under the contractile force of the elastic belt panels 16, 17 and the first leg elastics 26 should not be spaced away from the wearer's thighs during use of the diaper 10. In addition, with the arrangement such that the waist pocket panel 52 and the elastic belt panels 16, 17 are separately formed, itispossible to vary various conditions such as a distance dimension between the first waist elastics 44 and the second waist elastics 55 so that leakage prevention of body exudates from the rear waist region 12 by the contractile force of the first waist elastics 44 may be enhanced and an appropriate fit to the wearer's body may be ensured under the contractile force of the second waist elastics 55 while a desired air permeability is maintained through the elastic belt panels 16, 17. Additionally, since the second leg elastics 27 have curved portions 30 curving outwardly in the transverse direction X on the side of the rear waist region 12 so as to define spaced regions between the first leg elastics 26 and the second leg elastics 27, such spaced regions cooperate with spaced regions defined between the elastic belt panels 16, 17 and the waist pocket panel 52 and whereby the elastic effect of the first elastic region 40 to the first leg elastics 26 may be further inhibited.

The liquid-absorbent structure 15 is attached to the skin-facing surface of a main portion 59 of the outer cover 18 with a hot melt adhesive and includes an absorbent layer 60, abodyside liner 61 formedof a liquid-permeable sheet and covering at least the skin-facing surface of the absorbent layer and a leakage-barrier sheet 62 formed of a liquid-impermeable sheet and covering the non-skin-facing surface of the absorbent layer 60. Between the body side liner 61 and the absorbent layer 60, a plurality of thread, string or strand elastics 63 spaced apart from each other in the transverse direction X and extending in the longitudinal direction Y are arranged in a contractible state. Though not illustrated, the liquid-absorbent structure 15 is formed with a plurality of wrinkles/creases extending in the transverse direction X under the contraction of the elastics 63. The elastics 63 arranged on the liquid-absorbent structure 15 facilitate the diaper 10 as a whole to be stretched in the longitudinal direction Y and, even for the wearer having a relatively large circumferential waist size and in a supine posture, the first fastening elements 47 may be easily engaged with the second fastening element 48.

The absorbent layer 60 is formed of absorbent materials including a mixture of superabsorbent polymer particles (SAP) which is water-insoluble and capable of absorbing an amount of water 10 times or more of its own mass, wood fluff pulp and optionally a small quantity of thermoplastic fibers and wrapped with a liquid-permeable sheet such as tissue paper and a hydrophilic nonwoven fabric.

Referring to Figs. 4 and 5, in the front waist region 11, the waist pocket panel 51 is joined to the chassis 14 through a first joint region 71 extending in the transverse direction X on the skin-facing surface of the main portion 59 of the outer cover 18 to which the liquid-absorbent structure 15 is attached and second joint regions 72 extending in the longitudinal direction Y along the inner lateral edge portions 25a of the respective lateral elastic regions 25. Front end portions 74 of the respective lateral elastic regions 25 are joined to the main portion 59 of the outer cover 18 through the first joint region 71. A dimension L1 in the longitudinal direction Y of the first joint region 71 is smaller than a dimension L in the longitudinal direction Y of the waist pocket panel 51 and a central non-j oint region 75 is defined inboard of the first joint region 71 of the waist pocket panel 51 as viewed in the longitudinal direction Y and inboard of the second joint regions 72 of the waist pocket panel 51 as viewed in the transverse direction X. A front pocket 76 adapted to receive body exudates is defined between the central non-joint region 75 and the main portion 59 of the outer cover 18 and the front end portion of the liquid-absorbent structure 15.

Referring to Figs. 4, 6 and 7, in the rear waist region 12, the waist pocket panel 52 is joined to the chassis 14 through a first joint region 81 extending in the transverse direction X on the skin-facing surface of the main portion 59 of the outer cover 18 and second j oint regions 82 extending in the longitudinal direction Y along the inner lateral edge portions 25a of the respective lateral elastic regions 25. Rear end portions 84 of the respective lateral elastic regions 25 are joined to the main portion 59 of the outer cover 18 through the first joint region 81. A central non-joint region 85 is defined inboard of the first joint region 81 of the waist pocket panel 52 as viewed in the longitudinal direction Y and inboard of the second joint regions 82 of the waist pocket panel 52 as viewed in the transverse direction X. A rear space 86 adapted to receive body exudates is defined between the central non-joint region 85 and the main portion 59 of the outer cover 18 and the rear end portion of the liquid-absorbent structure 15.

In the rear waist region 12, the elastic belt panels 16, 17 are joined to the chassis 14 through third joint regions 83 extending in the longitudinal direction Y along the outer lateral edges 25b of the respective lateral elastic regions 25. The elastic belt panels 16, 17 are attached to the outer lateral edges 25b of the respective lateral elastic regions 25 and, consequently, during use of the diaper 10, the lateral elastic regions 25 are pulled in the transverse direction X under the effect of tensile strength of the elastic belt panels 16, 17 to put the inner lateral edge portions 25a in contact with the wearer's thighs, thereby forming leakage barriers 87 to be described later. The lateral elastic regions 25 respectively include lateral non-j oint regions 90 defined between the second and third joint regions 82, 83, respectively, and inboard of the first joint region 81 as viewed in the longitudinal direction Y.

Referring to Fig. 4, the dimension L1 of the waist pocket panel 51 in the longitudinal direction Y (common to the dimension of the waist pocket panel 52 in the longitudinal direction Y) is in a range of about 100 to about 200 mm, a dimension L2 in the longitudinal direction Y of the first joint region 71 in the front waist region 11 is in a range of about 50 to about 150 mm and a dimension L3 in the longitudinal direction Y of the first joint region 81 in the rear waist region 12 is in a range of about 20 to about 120 mm.

Referring to Fig. 8, during use of the diaper 10, the lateral elastic regions 25 are spaced away from the liquid-absorbent structure 15 toward the wearer's body and the inner lateral edge portions 25a of the respective lateral elastic regions 25 come in contact with the inner surface of the wearer's thighs to form the leakage barriers 87 so that the chassis 14 may have a generally U-shape in cross-section. When body exudates are voided onto the liquid-absorbent structure 15 of the diaper 10 during use of the diaper 10, the liquid-absorbent structure 15 is spaced away from the wearer's buttocks under its own weight and a relatively large volumetric body exudate receiving space 88 is formed between the wearer's buttocks and the liquid-absorbent structure 15. Particularly in the front and rear waist regions 11, 12, the front and rear pockets 76, 86 are opened and, in addition, the lateral non-joint regions 90 are defined in the rear waist region 12. In this way, the further large volumetric body exudates receiving space 88 is ensured. Portions of the lateral elastic regions 25 lying in front of the rear end portions 84 of the respective lateral elastic regions 25 and adapted to rise up lie outboard of the inner end edges 32 of the elastic belt panels 16, 17 as viewed in the longitudinal direction Y. With such arrangements, when the diaper 10 is put on the wearer's body, the lateral elastic regions 25 are pulled up by the elastic belt panels 16, 17 to rise up to form the relatively large leakage barriers 87 and allow further large amount of body exudates to be received. When a relatively large amount of body exudates is received and contained, the liquid-absorbent structure 15 is sufficiently spaced away from the wearer's buttocks and the wearer's buttocks should not be soiled with body exudates.

### <Second Embodiment>

Referring to Fig. 9, according to this embodiment, portions of the lateral elastic regions 25 intersecting with the transverse axis Q-Q are provided on the inner surface thereof with joint regions 89 through which the lateral elastic regions 25 are attached to the main portion 59 of the outer cover 18 with an adhesive or a welding technique. By providing these joint regions 89, the lateral elastic regions 25 are tucked and the midsection of the crotch region 13 has a narrow width. In consequence, the midsection of the crotch region 13 is put in contact with the wearer's body more tightly than the other region of the crotch region 13, whereby leakage of body exudates may be effectively prevented. A dimension of the respective joint regions 89 in the longitudinal direction Y may be set in a range of about 100 to about 400 mm.

### <Third Embodiment>

Referring to Fig. 10, according to this embodiment, the absorbent layer 60 of the liquid-absorbent structure 15 includes an absorbent material composed primarily of superabsorbent polymer particles, a first sheet (upper sheet) 91 lying on the skin-facing surface and formed from a liquid-permeable fibrous nonwoven fabric having a mass per unit area in a range of about 8.0 to about 15.0 g/m², preferably a mass per unit area of about 10.0 g/m² and a second sheet (lower sheet) 92 lying on the non-skin-facing surface and formed of a water-permeable or a low-water-permeable SMS fibrous nonwoven fabric having a mass per unit area in a range of about 8.0 to about 15.0 g/m², preferably a mass per unit area of 11.0 g/m².

The absorbent layer 60 further includes a plurality of generally elongate absorbent regions 93 defined by cells spaced apart from each other by a given dimension in the longitudinal direction Y and a seal region 94 substantially not provided with the absorbent material and surrounding the respective absorbent regions 93. In this regard, while the absorbent region 93 is divided into eight cells according to this embodiment, an area and the number of the cells of the absorbent region 93 may be appropriately varied depending on an absorption performance required for the liquid-absorbent structure 15 and, for example, the absorbent region 93 may be divided into eight or more cells or provided in the form of a single region extending over the entire liquid-absorbent structure 15.

In the absorbent region 93, superabsorbent polymer particles 95 having a mass per unit area in a range of about 30 to about 300 g/m², preferably a mass per unit area in a range of about 40 to about 280 g/m² are rather evenly secured to the inner surface of the first sheet 91 with a hot melt adhesive 96. In order to adjust the absorption rate of the absorbent layer 60 as a whole, for example, two types of superabsorbent polymer particles having different absorption rates may be used together. So long as advantageous effects of the present invention may be ensured, the absorbent materials may include, in addition to the superabsorbent polymer particles 95, various types of well known materials such as fluff pulp and optionally thermoplastic fibers at a relatively low mixing rate. Specifically, when the water-absorbent fibers such as fluff pulp is mixed, preferably about 0 to about 30% by mass of the absorbent material as a whole is mixed. While, as the hot melt adhesive 96, various types of well known adhesives maybe used, a hydrophobic adhesive is preferably used to prevent the superabsorbent polymer particles from falling off the first sheet 91 after the body exudates have been absorbed.

In the absorbent region 93, the first sheet 91 and the second sheet 92 are preferably partially joined to each other or not secured to each other. When the first and second sheets 91, 92 are partially joined to each other with the hot melt adhesive 96, it is possible to configure so that body exudates having flowed into the absorbent layer 50 are absorbed by the superabsorbent polymer particles 95 and joining between the first and second sheets 91, 92 is released due to swelling of the superabsorbent polymer particles 95. Meanwhile, in the seal region 94, more specifically, boundary regions between respective pairs of the adj acent absorbent cells 93 and the entire peripheral region of the absorbent layer 60, the first and second sheets 91, 92 are joined to each other with the hot melt adhesive 96. In this regard, the hot melt adhesive 96 is relatively densely distributed, i.e., in a planar pattern for the absorbent region 93 in order to secure the superabsorbent polymer particles 95 to the inner surface of the first sheet 91 while, in the seal region 94, the hot melt adhesive 96 is relatively sparsely distributed.

The absorbent material of the absorbent layer 60 is composed of only the superabsorbent polymer particles 95 and the sheet member wrapping them as has been described above and the absorbent material is thinner than where the absorbent material is composed of a mixture of the superabsorbent polymer particles and fluff pulp. This means that the absorbent materials flexibly conform to movements of the chassis. The first and second sheets 91, 92 are stably joined to each other in the seal region 94 to ensure a desired peel strength and a higher flexibility than where the first and second sheets 91, 92 are joined to each other in a whole area. In the absorbent region 93, the superabsorbent polymer particles 95 are evenly secured to the first sheet 91 and, in consequence, the superabsorbent polymer particles should not be unevenly distributed regardless of movements and postures of the wearer. While the seal region 94 is to seal the periphery of the absorbent region in order to prevent the movable superabsorbent polymer particles from falling off, a portion of the superabsorbent polymer particles 95 may sometimes creep into the seal region in the course of the manufacturing process in a range of mass per unit area smaller than a predetermined level. Though not illustrated, it is possible to arrange a plurality of elastics 63 extending in the longitudinal direction Y between the body side liner 61 and the absorbent layer 60 in a manner similar to the first embodiment.

The constituent elements of the diaper 10 are not limited to those described in the specification but the other various types of material widely used in the relevant technical field may be used without limitation unless otherwise stated. The terms "first", "second" and "third" used in the specification and Claims of the present invention are used merely to distinguish the similar elements, similar positions or the other similar means.

The disclosure of the present invention may be arranged in at least one or more of the following features.

A disposable diaper having a longitudinal direction and a transverse direction, the disposable diaper including:
a chassis including a skin-facing surface, a non-skin-facing surface, a front waist region, a rear waist region, a crotch region lying between the front and rear waist regions and a liquid-absorbent structure extending in the longitudinal direction at least in the crotch region; and
elastic belt panels extending outwardly in the transverse direction from lateral edges of the chassis in the rear waist region, wherein:
   the chassis further includes a main portion, lateral portions lying outboard of the main portion in the transverse direction provided with elastics and a pair of lateral elastic regions formed by folding the lateral portions inwardly; and
   at least in the rear waist region of the front and rear waist regions is provided with a waist pocket panel formed separately of the elastic belt panels and adapted to connect lateral edges of the lateral elastic regions and the elastic belt panels are attached to outer lateral edges of the lateral elastic regions.

The features of the present invention disclosed above may include at least the following embodiments:
(1) The lateral elastic regions and outer end portions of the waist pocket panel are secured to the main portion through a first joint region extending in the transverse direction and the waist pocket panel is attached to the skin-facing surface of the inner side portions of the lateral joint regions through second joint regions extending in the longitudinal direction and the waist pocket panel is provided with a non-joint region defined outboard of the first joint region as viewed in the longitudinal direction and inboard of the second joint regions as viewed in the transverse direction.
(2) The elastic belt panels are attached to the skin-facing surface of the outer lateral edges of the lateral elastic regions through third joint regions extending in the longitudinal direction and the lateral elastic regions include lateral non-joint regions lying between the second and third j oint regions and lying inboard of the joint region as viewed in the longitudinal direction.
(3) Each of the elastic belt panels has a first elastic region being elastically stretchable and contractible in the transverse direction and the waist pocket panel has a second elastic region being elastically stretchable and contractible in the transverse direction.
(4) A tensile stress per unit area of the first elastic region is higher than a tensile stress per unit area of the second elastic region.
(5) Each of the elastic belt panels has a first sheet lying on the side of the skin-facing surface, a second sheet lying on the side of the non-skin-facing surface and a plurality of thread, strand or string first waist elastics interposed between the first and second sheets and extending in the transverse direction; and the waist pocket panel has interior and exterior sheets and a plurality of thread, strand or string second waist elastics interposed between the interior and exterior sheets and extending in the transverse direction.
(6) Each of the elastic belt panels has the first elastic region and a inelastic tab lying outboard of the first elastic region as viewed in the transverse direction and an outer end portion of the tab is provided on the skin-facing surface with a fastening element adapted to be fastened to the exterior surface of the front waist region.
(7) Each of the leg elastics includes a plurality of thread, strand or string first leg elastics rectilinearly extending in the longitudinal direction along the inner lateral edge of the lateral elastic region and second leg elastics lying outboard of the first leg elastics as viewed in the transverse direction and the second leg elastics have rectilinear portions and curved portions gradually extending outwardly in the transverse direction as the second leg elastics extend rearwardly from the crotch region toward the rear waist region.
(8) Part of the lateral elastic regions is attached to the main portion in the crotch region.
(9) The liquid-absorbent structure has a liquid-permeable body side liner and an absorbent layer and, between the body side liner and the absorbent layer, a plurality of elastics extending in the longitudinal direction are arranged at least in a central portion of the absorbent layer.
(10) The liquid-absorbent structure has the liquid-permeable body side liner and the absorbent layer and the absorbent layer includes a liquid-permeable first sheet, a liquid-impermeable second sheet, superabsorbent polymer particles interposed between the first and second sheets, a non-absorbent region in which the first sheet and the second sheet are joined to each other and an absorbent region surrounded by the non-absorbent region in which the superabsorbent polymer particles are wrapped.

### {Reference Signs List}

- 10: disposable diaper
- 11: front waist region
- 12: rear waist region
- 13: crotch region
- 14: chassis
- 15: liquid-absorbent structure
- 16, 17: elastic belt panels
- 25: lateral elastic regions
- 25a: inner lateral edge portions of lateral elastic regions
- 25b: outer lateral edges of lateral elastic regions
- 26: first leg elastics
- 27: second leg elastics
- 29: rectilinear portions of second leg elastics
- 30: curved portions of second leg elastics
- 40: first elastic regions
- 42: first sheets
- 43: second sheets
- 44: first waist elastics
- 47: first fastening elements (fastening elements)
- 55: second waist elastics
- 56: second elastic region
- 59: main portion of chassis
- 60: absorbent layer
- 61: body side liner
- 63: elastics
- 91: first sheet
- 92: second sheet
- 93: absorbent regions
- 94: seal region
- 95: superabsorbent polymer particles
- X: transverse direction
- Y: longitudinal direction

## Claims

1. A disposable diaper (10) having a longitudinal direction (Y) and a transverse direction (X), the disposable diaper including:
a chassis (14) including a skin-facing surface, a non-skin-facing surface, a front waist region (11), a rear waist region (12), a crotch region (13)lying between the front and rear waist regions and a liquid-absorbent structure (15) extending in the longitudinal direction at least in the crotch region,
**characterised by** elastic belt panels (16, 17) extending outwardly in the transverse direction from lateral edges of the chassis in the rear waist region, wherein:
the chassis includes a main portion (59), lateral portions lying outboard of the main portion in the transverse direction provided with leg elastics (26, 27) and a pair of lateral elastic regions (25) formed by folding the lateral portions inwardly;
at least in the rear waist region of the front and rear waist regions is provided with a waist pocket panel (52) formed separately of the elastic belt panels and adapted to connect lateral edges of the lateral elastic regions and the elastic belt panels are attached to outer lateral edges of the lateral elastic regions;
the lateral elastic regions and outer end portions of the waist pocket panel are attached to the main portion through a first joint region (81) extending in the transverse direction and the waist pocket panel is attached to the skin-facing surface of the inner side portions of the lateral joint regions through second joint regions (82) extending in the longitudinal direction and the waist pocket panel is provided with a non-joint region (85) defined outboard of the first joint region as viewed in the longitudinal direction and inboard of the second joint regions as viewed in the transverse direction; and
the elastic belt panels are attached to the skin-facing surface of the outer lateral edges of the lateral elastic regions through third joint regions (83) extending in the longitudinal direction and the lateral elastic regions include lateral non-joint regions (90) lying between the second and third joint regions and lying inboard of the first joint region as viewed in the longitudinal direction.

2. The diaper according to claim 1, wherein each of the elastic belt panels has a first elastic region (40) being elastically stretchable and contractible in the transverse direction and the waist pocket panel has a second elastic region (56) being elastically stretchable and contractible in the transverse direction.

3. The diaper according to any one of claims 1 or 2, wherein:
each of the elastic belt panels has a first sheet (42) lying on the side of the skin-facing surface, a second sheet (43) lying on the side of the non-skin-facing surface and a plurality of thread, strand or string first waist elastics (44) interposed between the first and second sheets and extending in the transverse direction; and
the waist pocket panel has interior and exterior sheets (53, 54) and a plurality of thread, strand or string second waist elastics (55) interposed between the interior and exterior sheets and extending in the transverse direction.

4. The diaper according to any one of claims 1 through 3, wherein each of the elastic belt panels has the first elastic region and a inelastic tab lying outboard of the first elastic region as viewed in the transverse direction and an outer end portion of the tab is provided on the skin-facing surface with a fastening element (47) adapted to be fastened to the exterior surface of the front waist region.

5. The diaper according to any one of claims 1 through 4, wherein each of the leg elastics includes a plurality of thread, strand or string first leg elastics (26) rectilinearly extending in the longitudinal direction along the inner lateral edge of the lateral elastic region and second leg elastics (27) lying outboard of the first leg elastics as viewed in the transverse direction and the second leg elastics have rectilinear portions and curved portions gradually extending outwardly in the transverse direction as the second leg elastics extend rearwardly from the crotch region toward the rear waist region.

6. The diaper according to any one of claims 1 through 5, wherein part of the lateral elastic regions is attached to the main portion in the crotch region.

7. The diaper according to any one of claims 1 through 6, wherein the liquid-absorbent structure has a liquid-permeable body side liner (61) and a absorbent layer (60) and, between the body side liner and the absorbent layer, a plurality of elastics (63) extending in the longitudinal direction are arranged at least in a central portion of the absorbent layer.

8. The diaper according to any one of claims 1 through 6, wherein the liquid-absorbent structure has the liquid-permeable body side liner and the absorbent layer and the absorbent layer includes a liquid-permeable first sheet (91), a liquid-impermeable second sheet (92), superabsorbent polymer particles (95) interposed between the first and second sheets, a non-absorbent region (94) in which the first sheet and the second sheet are joined to each other and an absorbent region (93) surrounded by the non-absorbent region in which the superabsorbent polymer particles are wrapped.

## Patentansprüche

1. Einwegwindel (10), die eine Längsrichtung (Y) und eine Querrichtung (X) aufweist, wobei die Einwegwindel Folgendes einschließt:
einen Grundkörper (14), der Folgendes einschließt: eine der Haut zugewandte Oberfläche, eine nicht der Haut zugewandte Oberfläche, einen vorderen Taillenbereich (11), einen hinteren Taillenbereich (12), einen Schrittbereich (13), der zwischen dem vorderen und hinteren Taillenbereich liegt, und eine flüssigkeitsabsorbierende Struktur (15), die sich in der Längsrichtung zumindest in dem Schrittbereich erstreckt,
**gekennzeichnet durch** elastische Gürtelblenden (16, 17), die sich nach außen in der Querrichtung von den seitlichen Rändern des Grundkörpers in dem hinteren Taillenbereich erstrecken, wobei:
der Grundkörper Folgendes einschließt: ein Hauptteil (59), seitliche Teile, die außerhalb des Hauptteils in der Querrichtung liegen, mit elastischen Beinelementen (26, 27) und einem Paar an seitlichen elastischen Bereichen (25), die durch Falten der seitlichen Teile nach innen ausgebildet werden, bereitgestellt sind;
zumindest in dem hinteren Taillenbereich des vorderen und hinteren Taillenbereichs eine Taillentaschenblende (52) bereitgestellt ist, die separat von den elastischen Gürtelblenden ausgebildet ist und zur Verbindung der seitlichen Ränder der seitlichen elastischen Bereiche geeignet ist, und die elastischen Gürtelblenden an den äußeren seitlichen Rändern der seitlichen elastischen Bereiche angebracht sind;
die seitlichen elastischen Bereiche und äußeren Endteile der Taillentaschenblende an dem Hauptteil durch einen ersten Verbindungsbereich (81), der sich in der Querrichtung erstreckt, angebracht sind und die Taillentaschenblende an der der Haut zugewandten Oberfläche der inneren Seitenteile der seitlichen Verbindungsbereiche durch zweite Verbindungsbereiche (82), die sich in der Längsrichtung erstrecken, angebracht ist und die Taillentaschenblende mit einem Nicht-Verbindungsbereich (85) bereitgestellt ist, der außerhalb des ersten Verbindungsbereichs, in der Längsrichtung betrachtet, und innerhalb der zweiten Verbindungsbereiche, in der Querrichtung betrachtet, definiert ist; und
die elastischen Gürtelblenden an der der Haut zugewandten Oberfläche der äußeren seitlichen Ränder der seitlichen elastischen Bereiche durch dritte Verbindungsbereiche (83), die sich in der Längsrichtung erstrecken, angebracht sind und die seitlichen elastischen Bereiche seitliche Nicht-Verbindungsbereiche (90) einschließen, die zwischen den zweiten und dritten Verbindungsbereichen liegen und innerhalb des ersten Verbindungsbereichs, in der Längsrichtung betrachtet, liegen.

2. Windel nach Anspruch 1, wobei jede der elastischen Gürtelblenden einen ersten elastischen Bereich (40) aufweist, der in der Querrichtung elastisch dehnbar und zusammenziehbar ist, und die Taillentaschenblende einen zweiten elastischen Bereich (56) aufweist, der in der Querrichtung elastisch dehnbar und zusammenziehbar ist.

3. Windel nach einem der Ansprüche 1 oder 2, wobei:
jede der elastischen Gürtelblenden Folgendes aufweist: eine erste Lage (42), die auf der Seite der der Haut zugewandten Oberfläche liegt, eine zweite Lage (43), die auf der Seite der nicht der Haut zugewandten Oberfläche liegt, und eine Vielzahl von ersten elastischen Garn-, Faden- und Schnurtaillenelementen (44), die zwischen die erste und zweite Lage eingefügt sind und sich in der Querrichtung erstrecken; und
die Taillentaschenblende Folgendes aufweist: Innen- und Außenlagen (53, 54) und eine Vielzahl von zweiten elastischen Garn-, Faden- und Schnurtaillenelementen (55), die zwischen die Innen- und Außenlage eingefügt sind und sich in der Querrichtung erstrecken.

4. Windel nach einem der Ansprüche 1 bis 3, wobei jede der elastischen Gürtelblenden den ersten elastischen Bereich und eine unelastische Lasche aufweist, die außerhalb des ersten elastischen Bereichs, in der Querrichtung betrachtet, liegt, und ein äußeres Endteil der Lasche auf der der Haut zugewandten Oberfläche mit einem Befestigungselement (47), das zur Befestigung an der äußeren Oberfläche des vorderen Taillenbereichs geeignet ist, bereitgestellt ist.

5. Windel nach einem der Ansprüche 1 bis 4, wobei jedes der elastischen Beinelemente Folgendes einschließt: eine Vielzahl von ersten elastischen Garn-, Faden- und Schnurbeinelementen (26), die sich geradlinig in der Längsrichtung entlang des inneren seitlichen Rands des seitlichen elastischen Bereichs erstrecken, und zweite elastische Beinelemente (27), die außerhalb der ersten elastischen Beinelemente, in der Querrichtung betrachtet, liegen, und die zweiten elastischen Beinelemente Folgendes aufweisen: geradlinige Teile und gebogene Teile, die sich allmählich nach außen in der Querrichtung erstrecken, wenn sich die zweiten elastischen Beinelemente von dem Schrittbereich nach hinten zum hinteren Taillenbereich erstrecken.

6. Windel nach einem der Ansprüche 1 bis 5, wobei Teil der seitlichen elastischen Bereiche an den Hauptteil in dem Schrittbereich angebracht ist.

7. Windel nach einem der Ansprüche 1 bis 6, wobei die flüssigkeitsabsorbierende Struktur eine flüssigkeitsdurchlässige Körperseiteneinlage (61) und eine absorbierende Schicht (60) aufweist und zwischen der Körperseiteneinlage und der absorbierenden Schicht eine Vielzahl von elastischen Elementen (63), die sich in der Längsrichtung erstrecken, zumindest in einem mittleren Bereich der absorbierenden Schicht angeordnet ist.

8. Windel nach einem der Ansprüche 1 bis 6, wobei die flüssigkeitsabsorbierende Struktur die flüssigkeitsdurchlässige Körperseiteneinlage und die absorbierende Schicht aufweist und die absorbierende Schicht Folgendes einschließt: eine flüssigkeitsdurchlässige erste Lage (91), eine flüssigkeitsundurchlässige zweite Lage (92), superabsorbierende Polymerpartikel (95), die zwischen der ersten und zweiten Lage eingefügt sind, einen nicht-absorbierenden Bereich (94), in dem die erste Lage und die zweite Lage miteinander verbunden sind, und einen absorbierenden Bereich (93), umgeben von dem nicht-absorbierenden Bereich, in dem die superabsorbierenden Polymerpartikel umhüllt sind.

## Revendications

1. Couche jetable (10) ayant une direction allant dans le sens longitudinal (Y) et une direction allant dans le sens transversal (X), la couche jetable comprenant :
une armature (14) comprenant une surface orientée vers la peau, une surface non orientée vers la peau, une région avant au niveau de la taille (11), une région arrière au niveau de la taille (12), une région au niveau de l'entrejambe (13) reposant entre les régions avant et arrière au niveau de la taille et une structure absorbant les liquides (15) s'étendant dans la direction allant dans le sens longitudinal au moins dans la région au niveau de l'entrejambe,
**caractérisée par** des panneaux élastiques formant ceinture (16, 17) s'étendant vers l'extérieur dans la direction allant dans le sens transversal depuis des bords latéraux de l'armature dans la région arrière au niveau de la taille, dans laquelle :
l'armature comprend une partie principale (59), des parties latérales reposant à l'extérieur de la partie principale dans la direction allant dans le sens transversal comportant des élastiques pour les jambes (26, 27) et une paire de régions élastiques latérales (25) formées en pliant les parties latérales vers l'intérieur ;
au moins dans la région arrière au niveau de la taille parmi les régions avant et arrière au niveau de la taille se trouve un panneau formant poche au niveau de la taille (52) formé séparément des panneaux élastiques formant ceinture et adapté pour raccorder des bords latéraux des régions élastiques latérales et les panneaux élastiques formant ceinture sont attachés au niveau de bords latéraux extérieurs des régions élastiques latérales ;
les régions élastiques latérales et les parties d'extrémité extérieures du panneau formant poche au niveau de la taille sont attachées au niveau de la partie principale par le biais d'une première région d'assemblage (81) s'étendant dans la direction allant dans le sens transversal et le panneau formant poche au niveau de la taille est attaché au niveau de la surface orientée vers la peau des parties latérales intérieures des régions d'assemblage latérales par le biais de deuxièmes régions d'assemblage (82) s'étendant dans la direction allant dans le sens longitudinal et le panneau formant poche au niveau de la taille comporte une région de non-assemblage (85) définie à l'extérieur de la première région d'assemblage quand on regarde dans la direction allant dans le sens longitudinal et à l'intérieur de la deuxième région d'assemblage quand on regarde dans la direction allant dans le sens transversal ; et
les panneaux élastiques formant ceinture sont attachés au niveau de la surface orientée vers la peau des bords latéraux extérieurs des régions élastiques latérales par le biais de troisièmes régions d'assemblage (83) s'étendant dans la direction allant dans le sens longitudinal et les régions élastiques latérales comprennent des régions latérales de non-assemblage (90) reposant entre les deuxième et troisième régions d'assemblage et reposant à l'intérieur de la première région d'assemblage quand on regarde dans la direction allant dans le sens longitudinal.

2. Couche selon la revendication 1, dans laquelle chacun des panneaux élastiques formant ceinture a une première région élastique (40) qui est en mesure de s'étirer et de se contracter de manière élastique dans la direction allant dans le sens transversal et le panneau formant poche au niveau de la taille a une deuxième région élastique (56) qui est en mesure de s'étirer et de se contracter de manière élastique dans la direction allant dans le sens transversal.

3. Couche selon l'une quelconque des revendications 1 ou 2, dans laquelle :
chacun des panneaux élastiques formant ceinture a une première feuille (42) reposant sur le côté de la surface orientée vers la peau, une deuxième feuille (43) reposant sur le côté de la surface non orientée vers la peau et une pluralité de premiers élastiques au niveau de la taille de type fil, brin ou ficelle (44) intercalés entre les première et deuxième feuilles et s'étendant dans la direction allant dans le sens transversal ; et
le panneau formant poche au niveau de la taille a des feuilles intérieure et extérieure (53, 54) et une pluralité de deuxièmes élastiques au niveau de la taille de type fil, brin ou ficelle (55) intercalés entre les feuilles intérieure et extérieure et s'étendant dans la direction allant dans le sens transversal.

4. Couche selon l'une quelconque des revendications 1 à 3, dans laquelle chacun des panneaux élastiques formant ceinture a la première région élastique et une languette inélastique reposant à l'extérieur de la première région élastique quand on regarde dans la direction allant dans le sens transversal et une partie d'extrémité extérieure de la languette comporte sur la surface orientée vers la peau un élément de fixation (47) adapté à des fins de fixation sur la surface extérieure de la région avant au niveau de la taille.

5. Couche selon l'une quelconque des revendications 1 à 4, dans laquelle chacun des élastiques pour les jambes comprend une pluralité de premiers élastiques pour les jambes de type fil, brin ou ficelle (26) s'étendant de manière rectiligne dans la direction allant dans le sens longitudinal le long du bord latéral intérieur de la région élastique latérale et des deuxièmes élastiques pour les jambes (27) reposant à l'extérieur des premiers élastiques pour les jambes quand on regarde dans la direction allant dans le sens transversal et les deuxièmes élastiques pour les jambes ont des parties rectilignes et des parties courbes s'étendant progressivement vers l'extérieur dans la direction allant dans le sens transversal alors que les deuxièmes élastiques pour les jambes s'étendent vers l'arrière depuis la région au niveau de l'entrejambe vers la région arrière au niveau de la taille.

6. Couche selon l'une quelconque des revendications 1 à 5, dans laquelle une partie des régions élastiques latérales est attachée à la partie principale dans la région au niveau de l'entrejambe.

7. Couche selon l'une quelconque des revendications 1 à 6, dans laquelle la structure absorbant les liquides a une garniture côté corps perméable aux liquides (61) et une couche absorbante (60) et, entre la garniture côté corps et la couche absorbante, les élastiques d'une pluralité d'élastiques (63) s'étendant dans la direction allant dans le sens longitudinal sont agencés au moins dans une partie centrale de la couche absorbante.

8. Couche selon l'une quelconque des revendications 1 à 6, dans laquelle la structure absorbant les liquides a la garniture côté corps perméable aux liquides et la couche absorbante et la couche absorbante comprend une première feuille perméable aux liquides (91), une deuxième feuille imperméable aux liquides (92), des particules de polymère à fort pouvoir absorbant (95) intercalées entre les première et deuxième feuilles, une région non absorbante (94) dans laquelle la première feuille et la deuxième feuille sont assemblées l'une par rapport à l'autre et une région absorbante (93) entourée de la région non absorbante dans laquelle les particules de polymère à fort pouvoir absorbant sont enveloppées.
